(19) Europäisches Patentamt
European Patent Office
Office européen des brevets

(11) Numéro de publication: **0 069 010**
**B1**

(12) # FASCICULE DE BREVET EUROPEEN

(45) Date de publication du fascicule du brevet: **24.10.84**

(51) Int. Cl.³: **C 07 C 49/203**, C 07 C 49/587, C 07 C 45/51

(21) Numéro de dépôt: **82401147.2**

(22) Date de dépôt: **22.06.82**

(54) **Procédé de préparation de composés carbonyles delta-éthyléniques.**

(30) Priorité: **23.06.81 FR 8112301**

(43) Date de publication de la demande: **05.01.83 Bulletin 83/1**

(45) Mention de la délivrance du brevet: **24.10.84 Bulletin 84/43**

(84) Etats contractants désignés: **AT BE CH DE FR GB IT LI LU NL SE**

(56) Documents cités:
**JOURNAL OF THE AMERICAN CHEMICAL SOCIETY, vol. 102, no. 2, 16 janvier 1980 L.E. OVERMAN et al.: "Palladium(II) chloride catalyzed cope rearrangements of acyclic 1,5-dienes", pages 865-7**
**JOURNAL OF THE AMERICAN CHEMICAL SOCIETY, vol. 97, no. 16, 6 août 1975 D.A. EVANS et al.: "(3,3) Sigmatropic rearrangements of 1,5-diene alkoxides. The powerful accelerating effects of the alkoxide substituent", pages 4765-6**
**JOURNAL OF THE CHEMICAL SOCIETY, CHEMICAL COMMUNICATIONS, no. 22, 15 novembre 1978 Y. FUJITA et al.: "Effect of solvent on the oxy-cope rearrangement of the 4-isopropenyl-3,7-dimethylocta-1,6-dien-3-ol diastereomeric system", pages 972-3**

(73) Titulaire: **RHONE-POULENC SANTE, Les Miroirs 18 Avenue d'Alsace, F-92400 Courbevoie Cedex (FR)**

(72) Inventeur: **Bluthe, Norbert, 32, rue de la Baisse, F-69100 Villeurbanne (FR)**
Inventeur: **Gore, Jacques, 7, rue du Mont Cindre, F-69300 Caluire (FR)**
Inventeur: **Malacria, Max, 32, rue de la Baisse, F-69100 Villeurbanne (FR)**

(74) Mandataire: **Pilard, Jacques et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 069 010 B1**

ACTORUM AG

## Description

La présente invention concerne un procédé de préparation de composés carbonylés δ-éthyléniques de formule générale:

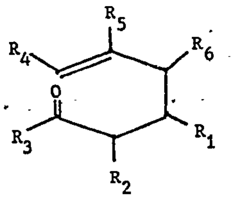

(I)

dans laquelle $R_1$, $R_4$ et $R_6$, identiques ou différents, représentent un atome d'hydrogène ou un radical hydrocarboné, $R_2$ représente un atome d'hydrogène, et $R_3$ et $R_5$, identiques ou différents, représentent chacun un radical hydrocarboné, $R_3$ et $R_4$ pouvant en outre représenter ensemble un radical alcoylène $(-CH_2-)_n$ dont un ou plusieurs atomes de carbone peuvent être éventuellement substitués par un ou plusieurs radicaux alcoyles contenant 1 à 4 atomes de carbone et dans lequel n est compris entre 3 et 20 inclusivement, par transposition d'un alcool diéthylénique de formule générale:

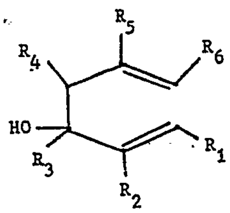

(II)

dans laquelle $R_1$, $R_2$, $R_3$, $R_4$, $R_5$ et $R_6$ sont définis comme précédemment.

Par radical hydrocarboné, on entend un radical acyclique contenant 1 à 20 atomes de carbone, dont la chaîne peut contenir une ou plusieurs doubles ou triples liaisons.

Les produits de formule générale (I) sont des intermédiaires particulièrement intéressants pour la préparation de produits ayant une activité biologique. Plus particulièrement, les produits de formule (I) trouvent leur application dans la synthèse de produits destinés à la pharmacie (vitamines A et E), l'agrochimie ou la parfumerie.

La transposition d'un alcool diéthylénique de formule générale (II) en un produit de formule générale (I) est une réaction connue appelée généralement transposition d'oxy-Cope. La transposition d'oxy-Cope a fait l'objet de nombreux travaux mais, compte tenu des conditions utilisées pour sa mise en œuvre, son intérêt pratique en a été très limité.

Par exemple, la thermolyse en phase vapeur selon le procédé décrit parr A. Viola et coll., «J. Amer. Chem. Soc.», *89*, 3462 (1967) est peu stéréosélective et elle conduit à des produits parasites de dégradation et de polymérisation dont l'origine est due aux hautes températures (voisines de 300°C) nécessaires au réarrangement.

Il a été montré, en particulier par Y. Fujita et coll., «Chem. Comm.», 972 (1978), et «Synthesis», 934 (1978), par M.L. Roumestant et coll., «Tetrahedron», *33*, 1283 (1977), et par A. Doutheau et coll., «Tetrahedron», *36*, 1953 (1980), que l'on augmentait le rendement et la stéréosélectivité en effectuant la réaction dans un solvant polaire aprotique, tel que la N-méthylpyrrolidone ou le diglyme, au reflux. Par ailleurs, D.A. Evans et A.M. Golob, «J. Amer. Chem. Soc.», *97*, 4765 (1975) ont montré que les alcoolates de potassium se transposaient plus facilement que les alcools eux-mêmes. Ainsi, certains alcools de formule générale (II) traités par l'hydrure de potassium dans le tétrahydrofuranne au reflux conduisent aux produits carbonylés δ-éthyléniques correspondants. Cependant, ces conditions ne conviennent pas pour la transposition de composés dont la stabilité en milieu très basique est faible.

Il est connu, d'après L.E. Overman, «J. Amer. Chem. Soc.», *102*, 865-867 (1980), de réaliser le réarrangement de diènes-1,5 substitués en -3 notamment par un radical phényle au moyen d'un catalyseur $PdCl_2\ (PhCN)_2$ par une transposition de Cope qui est différente d'une transposition d'oxy-Cope, mais la réaction décrite n'est pas d'application générale.

Il a maintenant été trouvé, et c'est ce qui fait l'objet de la présente invention, que la transposition d'oxy-Cope pouvait être réalisée à une température comprise entre −40°C et la température de reflux du mélange réactionnel en opérant en présence d'une quantité catalytique d'un complexe du palladium bivalent au sein d'un solvant convenable.

Selon l'invention, la transposition est avantageusement réalisée en présence d'un complexe du chlorure de palladium bivalent avec le benzonitrile, l'acétonitrile ou le cyclooctadiène dans un solvant organique tel que le tétrahydrofuranne, le chlorure de méthylène, le benzène, l'éther, à une température voisine de 20°C. La transposition peut également être mise en œuvre en présence de chlorure de palladium bivalent solubilisé par exemple par le chlorure de lithium sous forme de complexe $PdCl_4Li_2$ éventuellement formé *in situ*.

Généralement, le catalyseur est utilisé à raison de 0,01 à 0,15 mol par mole d'alcool diéthylénique mis en jeu.

La cétone δ-éthylénique de formule générale (I) peut être isolée, éventuellement après lavage à l'eau du mélange réactionnel, par application de méthodes physiques telles que la distillation ou la chromatographie.

Le complexe de palladium bivalent utilisé comme catalyseur peut être éventuellement régénéré et mis en œuvre pour la réalisation ultérieure de transposition d'oxy-Cope.

Le procédé selon la présente invention permet d'obtenir les cétones δ-éthyléniques avec de bons rendements qui peuvent cependant varier selon la nature de l'alcool diéthylénique mis en œuvre. Par ailleurs, le procédé est stéréospécifique et il conduit généralement à une proportion prépondérante de l'un des isomères.

Les exemples suivants, donnés à titre non limitatif, montrent comment l'invention peut être mise en pratique.

*Exemple 1:*

On maintient à une température voisine de 20° C, sous atmosphère inerte, un mélange de 0,126 g ($10^{-3}$ mol) de diméthyl-3,5 hexadiène-1,5 ol-3 et de 0,0153 g ($0{,}04 \cdot 10^{-3}$ mol) de palladium II-dichlorobis(benzonitrile) dans 7,5 cm³ de tétrahydrofuranne. Après 5 h de réaction, on ajoute 50 cm³ d'éther, puis le mélange réactionnel est lavé par 7 fois 10 cm³ d'eau. Après séchage de la couche organique sur sulfate de magnésium et évaporation du solvant sous pression réduite (20 mm de mercure; 2,7 kPa), on obtient 0,125 g de méthyl-6 heptène-6 one-2 dont les caractéristiques sont les suivantes:

– $P.E._{101,3\ kPa}$ = 142-144° C

– spectre infrarouge à partir d'un film liquide: bandes caractéristiques à 3070, 1720, 1650 et 890 $cm^{-1}$

– spectre de RMN ($CDCl_3$; δ en ppm, J en Hz): 1,65 (s, 3H); 1,7 à 2,0 (mt, 4H); 2,10 (s, 3H); 2,30 (t, J = 7,3H); 4,65 (s élargi, 2H).

Les caractéristiques de ce produit sont en accord avec celles qui ont été décrites par Viola et coll., «J. Amer. Chem. Soc.», *89,* 3462 (1967).

Le rendement est pratiquement quantitatif.

*Exemple 2:*

En opérant comme dans l'exemple 1, mais à partir de 0,262 g ($10^{-3}$ mol) d'isopropényl-4 triméthyl-3,7,11 dodécatriène 1,6,10 ol-3 et de 0,0575 g ($0{,}15 \cdot 10^{-3}$ mol) de palladium II-dichlorobis(benzonitrile), on obtient 0,223 g de triméthyl-6,10,14 pentadécatriène 6,9,13 one-2 dont les caractéristiques sont les suivantes:

– $P.E._{0,0013\ kPa}$ = 123-128° C

– spectre infrarouge à partir d'un film liquide: bandes caractéristiques à 3030, 1715, 1670, 1445 et 1160 $cm^{-1}$

– spectre de RMN ($CDCl_3$, δ en ppm, J en Hz): 1,61 et 1,63 (2 s élargis, 12H); 1,6 et 1,9 (mt, 2H); 1,9 à 2,15 (mt, 6H); 2,10 (s, 3H); 2,38 (t, J = 7, 2H); 2,63 (t; J = 7, 2H); 4,90 à 5,25 (mt, 3H).

Les caractéristiques spectrales de ce produit sont en accord avec celles décrites par Y. Fujita et coll., «Coll. Chem. Soc. Japan», *52,* 1983 (1979).

*Exemple 3:*

On maintient à une température voisine de 25° C, sous atmosphère inerte, un mélange de 0,126 g ($10^{-3}$ mol) de diméthyl-3,5 hexadiène-1, 5 ol-3 et de 0,0108 g ($0{,}04 \cdot 10^{-3}$ mol) de palladium II-dichlorobis(acétonitrile) dans 7,5 cm³ de tétrahydrofuranne. Après 2½ h de réaction, le tétrahydrofuranne est éliminé sous pression réduite (30 mm de mercure; 4 kPa). Après filtration sur une colonne de silice, en éluant avec de l'éther, on obtient 0,125 g de méthyl-6 heptène-6 one-2. Le rendement est pratiquement quantitatif.

*Exemple 4:*

On maintient à une température voisine de 20° C un mélange de 0,135 g ($7 \cdot 10^{-4}$ mol) d'isopropényl-4 diméthyl-3,7 octadiène-16 ol-3 et de 0,041 g de palladium II-dichlorobis(benzonitrile) dans 5 cm³ de tétrahydrofuranne. Après 2¾ h de réaction, le mélange réactionnel est lavé par 12 fois 5 cm³ d'eau permutée. Après séchage sur sulfate de magnésium et évaporation du solvant sous pression réduite (20 mm de mercure; 2,7 kPa), on obtient, avec un rendement de 76%, 0,103 g de diméthyl-6,10 undécadiène-6,9 one-2 dont les caractéristiques sont les suivantes:

– spectre infrarouge à partir d'un film liquide: bande caractéristique à 1720 $cm^{-1}$

– spectre de RMN ($CdCl_3$, δ en ppm, J en Hz): 1,5 à 1,8 (M, 11H); 1,95 (t, 2H); 2,07 (s, 3H); 2,34 (t, J = 7, 2H); 2,60 (dd, 2H); 4,8 à 5,3 (mt, 2H).

*Exemple 5:*

Une solution de 0,194 g ($10^{-3}$ mol) d'isopropényl-4 diméthyl-3,7 octadiène-1,6 ol-3 et de 0,0358 g ($0{,}93 \cdot 10^{-4}$ mol) de palladium II-dichlorobis(benzonitrile) dans 1 cm³ de benzène est agitée pendant 5 h sous atmosphère d'argon et à une température voisine de 20° C. Le mélange réactionnel est filtré sur une colonne de gel de silice (230-400 mesh) dont la hauteur est de 2 cm.

L'éluat est concentré, puis distillé sous pression réduite. On obtient ainsi 0,165 g d'un mélange contenant, d'après le dosage par chromatographie en phase vapeur, 91% de diméthyl-6,10 undécadiène-6,9 one-2 ($P.E._{0,0027\ kPa}$ = 150° C) dont les caractéristiques sont identiques à celles du produit de l'exemple 4.

Le rendement est de 77%.

*Exemple 6:*

Une solution de 0,194 g ($10^{-3}$ mol) d'isopropényl-4 diméthyl-3,7 octadiène-1,6 ol-3, de 0,0232 g ($1{,}3 \cdot 10^{-4}$ mol) de chlorure de palladium II et de 0,011 g ($2{,}6 \cdot 10^{-4}$ mol) de chlorure de lithium dans 1 cm³ de tétrahydrofuranne est agitée pendant 25 h sous atmosphère d'argon à une température voisine de 20° C.

Le mélange réactionnel est traité dans les conditions de l'exemple 5. On obtient ainsi 0,170 g d'un mélange contenant, d'après le dosage par chromatographie en phase vapeur, 80% de diméthyl-6,10 undécadiène-6,9 one-2 et de 5% d'alcool de départ non transformé.

Le rendement est de 70%.

*Exemple 7:*

Une solution de 0,194 g ($10^{-3}$ mol) d'isopropényl-4 diméthyl-3,7 octadiène-1,6 ol-3 et de 0,0332 g ($1{,}6 \cdot 10^{-4}$ mol) de palladium II-dichloro(cyclooctadiényle) dans 1 cm³ de tétrahydrofuranne est chauffée à reflux pendant 24 h sous atmosphère d'argon.

Le mélange réactionnel est traité dans les conditions de l'exemple 5. On obtient ainsi, avec un rendement pratiquement quantitatif, un mélange contenant, d'après le dosage par chromatographie en phase vapeur, 70% de diméthyl-6,10 undécadiène-6,9 one-2 et 19% d'alcool de départ non transformé.

*Exemple 8:*

On maintient à une température voisine de 20° C, sous atmosphère inerte, un mélange de 0,100 g ($0,4 \cdot 10^{-3}$ mol) d'isopropényl-2 vinyl-1 cyclododécanol-1 et de 0,007 g ($0,018 \cdot 10^{-3}$ mol) de palladium II-dichlorobis(benzonitrile) dans 5 $cm^3$ de tétrahydrofuranne. Après 6 h de réaction, on ajoute 75 $cm^3$ d'éther, puis le mélange est lavé par 5 fois 15 $cm^3$ d'eau. Après séchage sur sulfate de magnésium, filtration et évaporation du solvant sous pression réduite (20 mm de mercure; 2,7 kPa), on obtient 0,065 g de méthyl-5 cyclohexadécène-5 one-1 dont les caractéristiques sont les suivantes:

– spectre infrarouge à partir d'un film liquide: bande caractéristique à 1715 $cm^{-1}$

– spectre de RMN ($CDCl_3$, δ en ppm): 4,95-5,22 (m, 14H); 2,2-2,5 (m, 4H); 1,9-2,2 (m, 4H); 1,2-1,9 (m, 21H).

*Exemple 9:*

On maintient à une température voisine de 20° C, sous atmosphère inerte, un mélange de 0,166 g ($10^{-3}$ mol) d'isopropényl-2 vinyl-1 cyclohexanol-1 et de 0,038 g ($10^{-4}$ mol) de palladium II-dichlorobis(benzonitrile) dans 5 $cm^3$ de tétrahydrofuranne. Après 24 h de réaction, on ajoute 75 $cm^3$ d'éther, puis le mélange est lavé par 5 fois 15 $cm^3$ d'eau. Après séchage sur sulfate de magnésium, filtration et évaporation du solvant sous pression réduite (20 mm de mercure; 2,7 kPa), on obtient 0,070 g de méthyl-5 cyclodécène-5 one-1 dont les caractéristiques sont les suivantes:

– spectre infrarouge à partir d'un film liquide: bandes caractéristiques à 1710, 1100 $cm^{-1}$

– spectre de RMN ($CDCl_3$, δ en ppm): 1,47 (s, 3H); 1,55 à 2,15 (m, 10H); 2,15 à 2,37 (m, 2H); 2,37 à 2,62 (m, 2H); 5,2 (t, 1H, J = 7 Hz).

*Exemple 10:*

On maintient à une température voisine de 20° C, sous atmosphère inerte, un mélange de 0,090 g ($5 \cdot 10^{-4}$ mol) d'isopropényl-2 propényl-1 cyclohexanol-1 et de 0,008 g ($0,2 \cdot 10^{-4}$ mol) de palladium II-dichlorobis(benzonitrile) dans 5 $cm^3$ de tétrahydrofuranne. Après 6 h de réaction, on ajoute 75 $cm^3$ d'éther, puis le mélange est lavé par 5 fois 15 $cm^3$ d'eau. Après séchage par sulfate de magnésium, filtration et évaporation du solvant sous pression réduite (20 mm de mercure; 2,7 kPa), on obtient 0,050 g de produit de départ et 0,023 g de diméthyl-3,5 cyclodécène-5 one-1 dont les caractéristiques sont les suivantes:

– spectre infrarouge à partir d'un film liquide: bandes caractéristiques à 1705, 1100 $cm^{-1}$

– spectre de RMN ($CDCl_3$, δ en ppm): 0,97 (d, 3H, J = 7 Hz); 1,5 (s, 3H); 1,07 à 2,7 (m, 13H); 5,2 (t, 1H, J = 7 Hz).

## Revendications

1. Procédé de préparation de composés carbonylés δ-éthyléniques de formule générale:

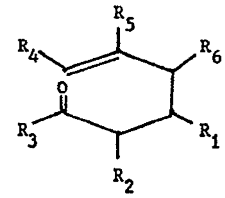

dans laquelle $R_1$, $R_4$ et $R_6$, identiques ou différents, représentent un atome d'hydrogène ou un radical hydrocarboné, $R_2$ représente un atome d'hydrogène, et $R_3$ et $R_5$, identiques ou différents, représentent un radical hydrocarboné, $R_3$ et $R_4$ pouvant en outre représenter ensemble un radical alcoylène $(-CH_2-)_n$ dont un ou plusieurs atomes de carbone peuvent être éventuellement substitués par un ou plusieurs radicaux alcoyles contenant 1 à 4 atomes de carbone et dans lequel n est compris entre 3 et 20 inclusivement, par transposition d'un alcool diéthylénique de formule générale:

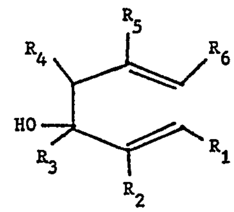

dans laquelle $R_1$, $R_2$, $R_3$, $R_4$, $R_5$ et $R_6$ sont définis comme précédemment, caractérisé en ce que l'on effectue la transposition à une température comprise entre −40° C et la température d'ébullition du mélange réactionnel en présence d'une quantité catalytique d'un complexe du palladium bivalent et isole le produit obtenu.

2. Procédé selon la revendication 1, caractérisé en ce que le complexe du palladium bivalent est choisi parmi le palladium II-dichlorobis(benzonitrile), le palladium II-dichlorobis(acétonitrile), le palladium II-dichloro(cyclooctadiényle) et le chlorure de palladium II sous une formule soluble.

3. Procédé selon la revendication 1, caractérisé en ce que l'on opère au sein d'un solvant organique.

4. Procédé selon la revendication 3, caractérisé en ce que l'on opère au sein du tétrahydrofuranne, du chlorure de méthylène, du benzène ou de l'éther.

5. Procédé selon la revendication 2, caractérisé en ce que le chlorure de palladium est solubilisé par le chlorure de lithium.

6. Procédé selon la revendication 1, caractérisé en ce que l'on met en œuvre un alcool diéthylénique de formule générale:

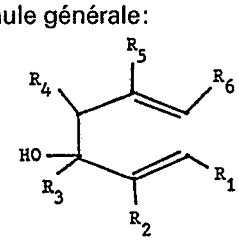

dans laquelle $R^1$, $R_4$ et $R_6$, identiques ou différents, représentent un atome d'hydrogène ou un radical alcoyle contenant 1 à 20 atomes de carbone, alcényle ou alcynyle contenant 2 à 20

atomes de carbone, $R_2$ représente un atome d'hydrogène, et $R_3$ et $R_5$, identiques ou différents, représentent chacun un radical alcoyle contenant 1 à 20 atomes de carbone, alcényle ou alcynyle contenant 2 à 20 atomes de carbone, $R_3$ et $R_4$ pouvant en outre représenter un radical alcoylène $(-CH_2-)_n$ dont un ou plusieurs atomes de carbone peuvent être éventuellement substitués par un ou plusieurs radicaux alcoyles contenant 1 à 4 atomes de carbone et dans lequel n est compris entre 3 et 20 inclusivement, étant entendu que les radicaux insaturés peuvent contenir plusieurs doubles ou triples liaisons.

## Patentansprüche

1. Verfahren zur Herstellung von δ-äthylenischen carbonylierten Verbindungen der allgemeinen Formel:

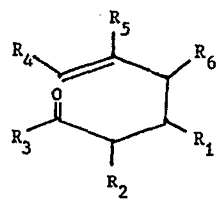

in welcher $R_1$, $R_4$ und $R_6$, die gleich oder voneinander verschieden sind, ein Wasserstoffatom oder einen Kohlenwasserstoffrest darstellen, $R_2$ ein Wasserstoffatom darstellt und $R_3$ und $R_5$, die gleich oder voneinander verschieden sind, einen Kohlenwasserstoffrest darstellen, $R_3$ und $R_4$ ausserdem zusammen einen Alkylenrest $(-CH_2-)_n$ darstellen können, der an einem oder mehreren Kohlenstoffatomen gegebenenfalls durch einen oder mehrere Alkylreste mit 1 bis 4 Kohlenstoffatomen substituiert sein kann, und worin n zwischen 3 und 20, einschliesslich, liegt, durch Umlagerung eines diäthylenischen Alkohols der allgemeinen Formel:

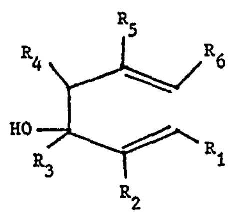

in welcher $R_1$, $R_2$, $R_3$, $R_4$, $R_5$ und $R_6$ die obige Bedeutung haben, dadurch gekennzeichnet, dass man die Umlagerung bei einer Temperatur zwischen −40°C und der Siedetemperatur der Reaktionsmischung in Gegenwart einer katalytischen Menge eines bivalenten Palladiumkomplexes ausführt und die erhaltene Verbindung isoliert.

2. Verfahren nach Anspruch 1, dadurch gekennzeichnet, dass der bivalente Palladiumkomplex ausgewählt ist aus Palladium(II)dichlorbis(benzonitril), Palladium(II)dichlorbis(acetonitril), Palladium(II)dichlor(cyclooctadienyl) und dem Palladium(II)chlorid in einer löslichen Form.

3. Verfahren nach Anspruch 1, dadurch gekennzeichnet, dass man in einem organischen Lösungsmittel arbeitet.

4. Verfahren nach Anspruch 3, dadurch gekennzeichnet, dass man in Tetrahydrofuran, Methylenchlorid, Benzol oder Äther arbeitet.

5. Verfahren nach Anspruch 2, dadurch gekennzeichnet, dass das Palladiumchlorid durch Lithiumchlorid solubilisiert wird.

6. Verfahren nach Anspruch 1, dadurch gekennzeichnet, dass man einen diäthylenischen Alkohol der allgemeine Formel:

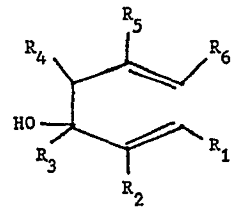

einsetzt, worin $R_1$, $R_4$ und $R_6$, die gleich oder von einander verschieden sind, ein Wasserstoffatom oder einen Alkylrest mit 1 bis 20 Kohlenstoffatomen, einen Alkenyl- oder Alkynylrest mit 2 bis 20 Kohlenstoffatomen darstellen, $R_2$ ein Wasserstoffatom darstellt und $R_3$ und $R_5$, die gleich oder voneinander verschieden sind, jeweils einen Alkylrest mit 1 bis 20 Kohlenstoffatomen, einen Alkenyl- oder Alkynylrest mit 2 bis 20 Kohlenstoffatomen darstellen, $R_3$ und $R_4$ ausserdem einen Alkylenrest $(-CH_2-)_n$ darstellen können, der an einem oder mehreren Kohlenstoffatomen gegebenenfalls durch einen oder mehrere Alkylreste mit 1 bis 4 Kohlenstoffatomen substituiert ist und worin n zwischen 3 und 20, einschliesslich, liegt, wobei die ungesättigten Reste selbstverständlich mehrere Doppel- oder Dreifachbindungen enthalten können.

## Claims

1. Process for the preparation of δ-ethylenic carbonyl compounds of the general formula:

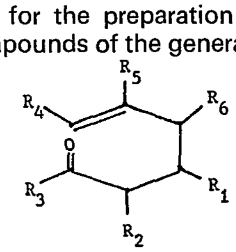

in which $R_1$, $R_4$ and $R_6$, which may be identical or different, represent a hydrogen atom or a hydrocarbon radical, $R_2$ represent a hydrogen atom and $R_3$ and $R_5$, which may be identical or different, represent a hydrocarbon radical, and $R_3$ and $R_4$ can furthermore conjointly represent an alkylene radical $(-CH_2-)_n$ of which one or more carbon atoms can optionally be substituted by one or more alkyl radicals containing 1 to 4 carbon atoms, and in which n is between 3 and 20 inclusive, by rearrangement of a diethylenic alcohol of the general formula:

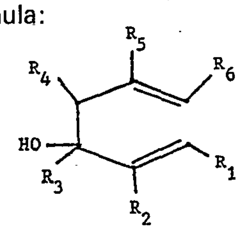

in which $R_1$, $R_2$, $R_3$, $R_4$, $R_5$ and $R_6$ are defined as above, characterised in that the rearrangement is effected at a temperature of between −40°C and the boiling point of the reaction mixture in the presence of a catalytic amount of a complex of bivalent palladium, and the product obtained is isolated.

2. Process according to Claim 1, characterised in that the complex of bivalent palladium is chosen from amongst palladium(II)dichloro-bis-(benzonitrile), palladium(II)dichloro-bis-(acetonitrile), palladium(II)dichloro-(cyclooctadienyl) and palladium(II)chloride in a soluble form.

3. Process according to Claim 1, characterised in that it is carried out in an organic solvent.

4. Process according to Claim 3, characterised in that it is carried out in tetrahydrofuran, methylene chloride, benzene or ether.

5. Process according to Claim 2, characterised in that the palladium chloride is rendered soluble with lithium chloride.

6. Process according to Claim 1, characterised in that a diethylenic alcohol of the general formula:

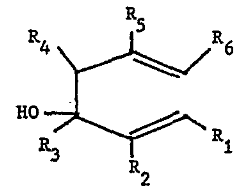

is used, in which $R_1$, $R_4$ and $R_6$, which may be identical or different, represent a hydrogen atom or an alkyl radical containing 1 to 20 carbon atoms or an alkenyl or alkinyl radical containing 2 to 20 carbon atoms, $R_2$ represents a hydrogen atom and $R_3$ and $R_5$, which may be identical or different, each represent an alkyl radical containing 1 to 20 carbon atoms or an alkenyl or alkinyl radical containing 2 to 20 carbon atoms and $R_3$ and $R_4$ may furthermore represent an alkylene radical $(-CH_2-)_n$ of which one or more carbon atoms can optionally be substituted by one or more alkyl radicals containing 1 to 4 carbon atoms, and in which n is between 3 and 20 inclusive, it being understood that the unsaturated radicals may contain a plurality of double or triple bonds.